Europäisches Patentamt

European Patent Office

Office européen des brevets

(19)

(11) Veröffentlichungsnummer: **0 040 838**
**A1**

(12) EUROPÄISCHE PATENTANMELDUNG

(21) Anmeldenummer: **81103955.1**

(22) Anmeldetag: **22.05.81**

(51) Int. Cl.³: **A 61 B 5/10**

(30) Priorität: **22.05.80 US 152513**

(43) Veröffentlichungstag der Anmeldung: **02.12.81**
**Patentblatt 81/48**

(84) Benannte Vertragsstaaten: **AT BE CH DE FR GB IT LI NL SE**

(71) Anmelder: **SIEMENS AKTIENGESELLSCHAFT Berlin und München, Postfach 22 02 61, D-8000 München 22 (DE)**

(72) Erfinder: **Rüll, Hartwig, Dr., Balduin-Helm-Strasse 49, D-8080 Fürstenfeldbruck (DE)**
Erfinder: **Feix, Wolfgang, 812 A Country Club Pkwy, Mt.Laurel New Jersey 08054 (US)**

(54) **Fingerabdruckwandler und Verfahren zum Analysieren eines Fingerabdruckes.**

(57) Es wird ein Fingerabdruckwandler beschrieben, der ein Abtastelement (2) umfaßt, das eine Abtastfläche (3) zur Aufnahme eines Fingerabdrucks (8) aufweist. Das Abtastelement (2) ist aus einem Material gefertigt, welches eine Übergangs- oder Erweichungstemperatur aufweist, die höher als die Umgebungstemperatur und in der Nähe der Temperatur des Fingers liegt. Die Erweichungstemperatur ist insbesondere so gewählt, daß das Material des Abtastelements (2) an der Oberfläche zu schmelzen beginnt, wenn der Finger auf die Abtastfläche (3) gedrückt wird. Die Abtastfläche (3) weist ein Oberflächenrelief zufälliger räumlicher Verteilung auf. Anders ausgedrückt: die Abtastfläche (3) weist verschiedene Erhebungen auf, die kein spezielles Muster zeigen. Es wird auch ein Verfahren zur Analysierung von Fingerabdrücken beschrieben, welches folgende Verfahrensschritte aufweist: Auf einer Abtastfläche (3) eines Abtastelements (2) wird ein Oberflächenrelief mit zufälliger räumlicher Verteilung erzeugt. Auf der Abtastfläche (3) wird durch Aufdrücken eines Fingers ein Fingerabdruck erzeugt. Der Fingerabdruck wird aus der Abtastfläche (3) optisch ausgelesen.

-1-

SIEMENS AKTIENGESELLSCHAFT
Berlin und München

Unser Zeichen
VPA 80 P 8217 E

Fingerabdruckwandler und Verfahren zum Analysieren eines Fingerabdrucks

Die vorliegende Erfindung bezieht sich auf einen Fingerabdruckwandler nach dem Oberbegriff des Patentanspruchs 1 und auf ein Verfahren zum Analysieren eines Fingerabdrucks.

In einem Fingerabdruck-Eingabewandler oder -sensor wird der zu überprüfende Finger gewöhnlich gegen eine flache Oberfläche gedrückt, beispielsweise auf eine Seite einer Glasplatte und das aus Rippen und Furchen bestehende Muster des Fingers wird durch Abtasteinrichtungen, beispielsweise einen abfragenden Lichtstrahl abgetastet. Die Verarbeitung der auf diese Weise erhaltenen Fingerabdruckinformation kann Lasertechniken umfassen. Derartige Vorrichtungen zur Identifizierung von Fingerabdrücken werden im allgemeinen zur Kontrolle des Zugangs von Individuen zu Informationen (Informationszugangskontrolle), beispielsweise zu Rechnerendgeräten (Informationszugangskontrolle) oder zu Gebäuden (physische Zugriffskontrolle) benutzt.

Eines der im Zusammenhang mit Fingerabdrucksensoren stehenden Probleme betrifft die zuverlässige Abtastung schwacher Fingerabdrücke. "Schwache Fingerabdrücke" sind Fingerabdrücke, die eine geringe Modulationstiefe auf der Abtastfläche aufweisen oder anders ausgedrückt, einen geringen topografischen Reliefwert. Ein schwacher Fingerabdruck kann auftreten, wenn der zu prüfende Finger nicht stark gegen die Abtastfläche gedrückt wird, wenn der Finger nicht genügend Flüssigkeit, beispielsweise Öl oder Feuchtigkeit, aufweist und dadurch keine ausreichen-Ed 1 Sti/25.3.81

dem Spuren auf der Fläche hinterläßt, usw.. Es wird deshalb ein Fingerabdruck-Eingabesensor oder -wandler benötigt, der so ausgebildet ist, daß er nicht nur Fingerabdrücke regulärer Qualität, sondern auch schwache Fingerabdrücke geringer Modulationstiefe abtasten kann. Es besteht auch ein Bedarf für ein Verfahren zur leichten Analysierung von Fingerabdrücken.

Allgemein gesprochen sollte der Fingerabdruckwandler die Übertragung der in der Struktur eines menschlichen Fingerabdrucks enthaltenen Information in ein Endgerät zur Verifikation ermöglichen, so daß ein maximaler in der Hautstruktur enthaltener Informationsbetrag erlangt werden kann. Der Fingerabdruckwandler sollte schnell arbeiten und nach jedem Analysierungs- oder Verifikationsversuch wiederbenutzbar sein.

Aus der US-PS 4 053 228 geht ein Fingeridentifizierungsproblem hervor, in welchem ein Fingerabdruck dadurch erzeugt wird, daß ein Finger gegen die Rückfläche einer transparenten Glasplatte gedrückt und der Finger in einer vorbestimmten Lage darauf gehalten wird. Dieser Fingerabdruck wird von einem, durch die Vorderfläche der Glasplatte gestrahlten Lichtstrahl abgetastet oder abgefragt. Der abfragende Strahl wird teilweise an der Rückfläche der Glasplatte reflektiert und dadurch ein Signalstrahl erzeugt, welcher Fingerabdruckinformation trägt. Die Rückfläche der Glasplatte ist mit einem Überzug überdeckt, welcher dazu dient, den Unterschied im Reflexionsvermögen zwischen den Furchen und Rippen des Fingerabdrucks zu verstärken. Insbesondere verstärkt der Überzug auf der Rückfläche den Unterschied zwischen dem Reflexionsvermögen auf dieser Fläche zwischen jenen Feldern, in denen die Rippen des Fingers in innigem Kontakt mit der Rückfläche der Platte stehen und jenen Feldern unter den Furchen des Fingers, in denen Luft mit der Rückfläche der Platte in Kontakt steht. In diesem Fingerabdruckwandler wird ein verstärkter Fingerabdruck nur so lange erzeugt, wie der Finger die Rückfläche der Platte berührt und dies ist kurzzeitig.

Aus der US-PS 4 120 585 ist ein nachgiebiges elastisches Prisma zum Gebrauch in einem optischen Abbildungssystem z.B. einem Fingerabdruckleser, bekannt geworden. Die Basisfläche des Prismas wird von dem zu untersuchenden Finger berührt. Das nachgiebige Prisma verformt sich unter dem Druck des berührenden Fingers. Licht aus einer Lichtquelle durchstrahlt eine Seitenfläche des Prismas in Richtung Basisfläche, wo es reflektiert und durch die andere Seitenfläche in Richtung eines lichtempfindlichen Elements strahlt. Dieses Element ist zum Auslösen oder Betätigen des optischen Abbildungssystems vorgesehen. Wenn ausreichender Fingerabdruck vorhanden ist, wird zwischen der Basisfläche und einem Gehäuse ein Luftspalt erzeugt, der in einem zusätzlichen Feld resultiert, dessen Lichtstrahl zum lichtempfindlichen Element gerichtet wird. Dieses Element aktiviert das System, in dem es einen Schalter schließt. Bei diesem Fingerabdrucksystem werden keine Einrichtungen zum Verarbeiten schwacher Fingerabdrücke erörtert.

Aus STIE, Vol. 185, Optical Processing Systems (1979) S. 86 - 92 ist eine elastomere Speichervorrichtung bekannt geworden, die einen Fotoleiter benutzt. Ein Glassubstrat wird zur Bildung einer transparenten leitenden Grundebene chemisch geätzt. Die Grundebene wird mit dem erwähnten Fotoleiter bedeckt und mit einer dünnen elastomeren Schicht überzogen.

Mehrere 10 µm (mehrere mils) über der elastomeren Oberfläche und parallel dazu ist eine Ladungsfläche, insbesondere ebene angeordnet. Der daraus resultierende
Spalt ist mit Argon unter niedrigem Druck gefüllt.
Während des Betriebes ist zwischen der Ladungsfläche
und der Grundfläche eine Hochspannungsquelle geschaltet,
die eine quer zu der elastomerischen oder fotoleitenden
Schicht gerichtete elektrostatische Kraft erzeugt. Die
elektrostatische Kraft steht senkrecht auf der elastomerischen Oberfläche und ist in jedem Punkt der elektrischen Feldstärke proportional. Weil das Elastomer inkompressibel und die Kraft gleichmäßig ist, tritt keine
Verformung auf. Wenn der Fotoleiter einer Lichtverteilung
ausgesetzt wird, werden Ladungsträgerfotos erzeugt und
bewegen sich in dem elektrischen Feld in Richtung zur
Grenzfläche zwischen Elastomer und Fotoleiter, wo sie
aufgefangen werden. Die aufgefangenen Ladungen bilden
eine elektrostatische Oberflächenladungsschicht, in
welcher die Ladungsdichteverteilung direkt proportional
zur Belichtungsverteilung des Eingangssignals ist. Die
resultierende elektrische Feldverteilung bewirkt eine
Verformung des Elastomers, wobei ein Oberflächenreliefmuster erzeugt wird, welches in direktem Bezug zur Belichtungsverteilung durch die Oberflächenladungsdichte
steht. Die Verformung setzt sich so lange fort, bis die
ungleichmäßige Ladungsverteilung beibehalten wird. Diese
Speichervorrichtung ist übrigens nicht zur Verwendung als
ein Fingerabdruckwandler in Betracht gezogen worden.

Aus SPIE, Vol. 123, Optical Storage Materials and
Methods (1977) S. 32 - 36 ist ein thermoplastisches
Datenspeichermedium bekannt geworden. Dieses Medium
ist eine mehrschichtige Vorrichtung, die aus einer
thermoplastischen Schicht, einer fotoleitenden Schicht
und einer transparenten leitenden Schicht auf einem
Glassubstrat oder flexiblen Polyestersubstrat besteht.
Die optische Datenspeicherung in Thermoplasten basiert auf dem Prinzip, daß sich ein Thermoplast unter Druck verformt, wenn er auf eine geeignete Temperatur erhitzt wird. Während des Einschreibens wird eine gleichmäßige Ladung auf der thermoplastischen Oberfläche erzeugt, dann wird die Vorrichtung mit einem holografischen Muster belichtet, welches die Leitfähigkeit des Fotoleiters und folglich die Oberflächenladungsverteilung verändert. Dann wird die Vorrichtung mit einem optischen Muster belichtet, welches die Leitfähigkeit des Fotoleiters und folglich die Oberflächenladungsverteilung verändert. Diese ungleichmäßige Ladungsverteilung hat elektrostatische Kräfte zur Folge, welche den Thermoplasten bei Erwärmung auf eine kritische Erweichungs- oder Entwicklungstemperatur verformen. Wenn die Probe abkühlt, bleibt die dem optischen Muster entsprechende Verformung erhalten und die Information kann durch Beleuchtung mit einem Auslesestrahl wiedergegeben werden. Auch dieses Speichermedium ist für eine Anwendung als ein Fingerabdruckwandler nicht vorgesehen.

Ähnliche optische Aufzeichnungs- und Speichervorrichtungen sind aus SPIE, Vol. 123, Optical Storage Materials and Methods (1977) S. 10 - 16 und S. 74 - 77 bekannt geworden.

Der Erfindung liegt die Aufgabe zugrunde, einen Fingerabdruckeingabesensor oder -wandler einfachen Aufbaus anzugeben, welche eine beträchtliche Menge von in der Hauptstruktur eines menschlichen Fingerabdrucks enthaltenen Information festzustellen und abzufühlen vermag bzw. ein Verfahren zur Analysierung eines Fingerabdrucks auf einer Oberfläche anzugeben.

Diese Aufgabe wird durch die im kennzeichnenden Teil des Patentanspruchs 1 bzw. 10 angegebenen Merkmale gelöst.

Bevorzugte und vorteilhafte Ausführungsformen der Erfindung gehen aus den Unteransprüchen 2 bis 9 bzw. 11 und 12 hervor.

Vorteile der Erfindung sind darin zu sehen, daß der erfindungsgemäße Fingerabdruckwandler bzw. das erfindungsgemäße Verfahren schnell arbeitet, nach einer vorangegangenen Fingerabdruckuntersuchung wieder benutzt werden kann, ein Fingerabdruck für eine gewisse Zeitdauer gespeichert werden kann und ein Fingerabdruck in Transmission oder Reflexion optisch ausgelesen werden kann.

Der erfindungsgemäße Fingerabdruckeingabewandler umfaßt gemäß den Ansprüchen ein Abtastelement, das eine Abtastfläche zur Aufnahme eines Fingerabdrucks aufweist. Die Abtastfläche weist ein Oberflächenrelief mit zufälliger Raumfrequenzverteilung auf. Anders ausgedrückt: Es gibt Variationen in den Erhebungen auf oder der Oberfläche (= Relief), die kein spezifisches Muster aufweisen ( = zufällig). D.h., daß es eine Vielzahl von Elementen oder Punkten gibt, welche einem spezifizierten geometrischen Postulat genügen, nämlich einer Zufallsverteilung über der Abtastfläche. Diese Erhebungen, Elemente oder Punkte bestehen vorzugsweise aus dem gleichen Material, wie das Abtastelement. Sie sind klein im Vergleich zum Fingerabdruck. Ihre Größe ist so gewählt, daß eine beträchtliche Lichtstreuung beobachtet werden kann, wenn die Oberfläche beleuchtet wird. Das Abtastelement ist aus einem Material gefertigt, welches eine Übergangs- oder Erweichungstemperatur aufweist, die höher als die Umgebungstemperatur ist und nahe bei der Temperatur des Fingers oder menschlichen Körpers liegt. Insbesondere wird die Erweichungstemperatur so ausgewählt, daß das Material des Elements zu schmelzen beginnt, wenn der Finger auf die Abtastfläche gedrückt wird. Dieser Schmelzprozeß entsteht aufgrund des auf die Oberfläche durch den
Finger ausgeübten Druckes und/oder der ausgeübten
Wärme. Im allgemeinen sollte die Erweichungstemperatur
des Abtastelements etwa 40°C betragen oder darüber
liegen. Vorzugsweise liegt die Erweichungstemperatur
nur einige wenige Celsiusgrade über der Temperatur
des menschlichen Körpers.

Das Abtastelement kann aus einem elastischen oder weichen Material gefertigt sein. Vorzugsweise ist es aus
einem elastomerischen oder thermoplastischen Material
gefertigt, beispielsweise aus plastifiziertem Polystyrol.
Es kann auch aus einem Material gerfertigt sein, dessen
Eigenschaften denen eines elastomerischen Materials
ähnlich sind. Unter einem elastomerischen Material
ist irgendeines aus jenen verschiedenen Polymeren zu
verstehen, welche die elastischen Eigenschaften von
natürlichem Gummi aufweisen. Insbesondere kann ein Elastomer mit vergleichsweise niedriger Erweichungstemperatur gewählt werden. Thermoplaste sind Polymere, die
durch Wärme wiederholt erweichbar (schmelzbar) und durch
Abkühlen härtbar sind. Typische Thermoplaste sind
Polyäthylen, Polypropylen, Polystyrole, Polyacryle,
Polyvenyle, Nylonstoffe und Fluorpolymere.

Um einen Fingerabdruck auf der Abtastfläche auszulöschen
und das zufällige Relief wieder herzustellen, kann eine
Einrichtung zum Erzeugen einer elektrostatischen Aufladung auf der Abtastfläche vorgesehen sein, sowie eine
Heizeinrichtung zum Erwärmen der Abtastfläche auf dem
Abtastelement auf eine Temperatur, die zumindest der
Erweichungstemperatur des Materials entspricht, aus dem
das Abtastelement gefertigt ist.

Die oben erwähnte Aufladung kann auf der Oberfläche,
vorzugsweise durch eine Koronabeladung, erzeugt werden.
Zu diesem Zweck kann die Aufladungseinrichtung einen Draht enthalten, der in einem Abstand von der Oberfläche des Abtastelements angeordnet ist, sowie eine
Spannungsquelle zum Anlegen einer Gleichspannung zwischen dem Draht und der Oberfläche des Abtastelements.
Die Ladeeinrichtung kann auch eine flächige Elektrode
in Form eines Drahtgitters umfassen, welches nahe
bei der Oberfläche des Abtastelements angeordnet ist,
sowie eine Spannungsquelle zum Anlegen einer gleichspannung zwischen der Elektrode und dem Abtastelement.

Die oben erwähnte Heizeinrichtung kann eine elektrische
Heizeinrichtung sein. Sie kann einen Heizwiderstand
enthalten, der beispielsweise ein Widerstandsdraht,
ein flächiges Drahtgitter oder eine transparente Elektrode sein kann, die einen geeigneten Flächenwiderstand
aufweist. Im Betrieb fließt durch den Heizwiderstand
ein Heizstrom. Das Drahtgitter kann in Bezug auf die
Wellenlänge des abtastenden Lichtstrahls transparent
sein. Zur Erzielung einer guten Wärmekopplung kann der
Heizwiderstand an dem Abtastelement angebracht sein.

Die Heizeinrichtung braucht jedoch nicht notwendig
eine ohmsche Heizeinrichtung oder Widerstandsheizung
zu sein. Die Erwärmung kann auch durch Strahlung erreicht werden. Beispielsweise kann Wärme zur Abtastfläche mittels eines Infrarot-(IR)-Laser, insbesondere
einer IR-LED, mittels eines Halbleiterlasers oder mittels
ähnlicher IR-Lichtquellen übertragen werden.

Die in dem Abtastelement gespeicherte Fingerabdruckinformation kann entweder durch optische Einrichtungen
beispielsweise in Totalreflexion oder durch Laseroptiken
in bekannter Weise ausgelesen werden. Das topografische
Relief kann in irgendeiner bekannten Weise analysiert
werden.

Im Betrieb des Eingabewandlers wird in einem ersten Schritt ein Oberflächenrelief zufälliger Raumfrequenzverteilung und zufälliger Orientierung auf einer Abtastfläche eines Abtastelements erzeugt. Dann wird in einem zweiten Schritt der zu untersuchende Finger gegen die Oberfläche des Abtastelements gedrückt, so daß er ein Oberflächenrelief zurückläßt. Aufgrund der Temperatur und des Drucks des Fingers schmilzt das zufällige Oberflächenrelief lokal, vorzugsweise an Stellen, wo die Rippen oder Erhebungen des Fingers das Abtastelement berühren. An diesen Stellen wird die zufällige Verteilung der Erhebungen zerstört. Die Folge davon ist, daß die geschmolzenen Teile kein Licht mehr in der gleichen Weise streuen als die unzerstörte Umgebung. Das bedeutet, daß der Fingerabdruck vom Wandler mittels optischen Mitteln leicht gelesen werden kann.

Zur Auslöschung des Reliefs wird die Abtastfläche erwärmt und gleichzeitig elektrostatisch aufgeladen. Dann wird die Fläche unter die Erweichungstemperatur abgekühlt. Beispielsweise kann ein Heizstrom durch den an das elastomerische Material wärmegekoppelten Heizwiderstand geleitet werden. Beim Erwärmen der Abtastfläche wird diese weich und die geschmolzene Fläche plättet sich aufgrund der Oberflächenspannung. Wegen der Beladung wird jedoch ein Zufallsrelief erzeugt. Der Wandler wird auf diese Weise für einen neuen Fingerabdruck-Identifizierungszyklus vorbereitet.

Einer der Vorteile der vorliegenden Erfindung liegt darin, daß das verstärkte Relief auf der Oberfläche des Abtastelements stabil oder "eingefroren" bleibt, nachdem der Finger gegen die Abtastfläche gedrückt worden ist.

Bevorzugte Ausführungsformen der Erfindung werden anhand der Figuren in der nun folgenden Beschreibung, aus der neben den genannten Vorteilen noch weitere Vorteile hervorgehen, näher erläutert. Von den Figuren zeigen:

Figur 1 einen Querschnitt durch eine Ausführungsform
eines erfindungsgemäßen Fingerabdruckwandlers;

Figur 2 eine Draufsicht den in Figur 1 dargestellten
Fingerabdruckwandler, wobei eine zufällige
Erhebungsverteilung auf der Abtastfläche dargestellt ist;

Figur 3 den in Figur 1 dargestellten Querschnitt mit
einem auf die Abtastfläche gedrückten Finger;

Figur 4 wieder eine Draufsicht auf den Fingerabdruckwandler nach Figur 1, wobei ein Fingerabdruck
auf der Abtastfläche dargestellt ist;

Figur 5 in vergrößerter Darstellung einen Querschnitt
des Fingerabdruckwandlers gemäß Figur 4 mit
weggebrochenen Teilen, wobei geriffelte Elemente
und an den Stellen, wo der Fingerabdruck gemacht
wurde, flache Elemente auf der Abtastfläche
gezeigt sind;

Figur 6 eine Anordnung zur Gewinnung oder zum Lessen des
Fingerabdrucks;

Figur 7 eine Vorrichtung zum Auslöschen des Fingerabdrucks und zur Wiedergewinnung des räumlich
zufälligen Reliefs;

Figur 8 einen Querschnitt durch einen Fingerabdruckwandler, der für eine nächste Überprüfung bereit ist;

Figur 9 einen erfindungsgemäßen Fingerabdruckwandler, der
mittels einer IR-Lichtquelle erwärmbar ist;

0040838

-11-     VPA 80 P 8217  E

Figur 10 **eine** Draufsicht auf eine Elektrode, welche in
einem erfindungsgemäßen Wandler benutzt werden
kann;

Figur 11 eine perspektivische Darstellung einer Ausführungsform der Erfindung, welche eine gitterförmige Beladungselektrode aufweist;
und

Figur 12 eine perspektivische Darstellung einer anderen
Ausführungsform der Erfindung, welche einen
einzelnen Draht als Beladungselektrode aufweist.

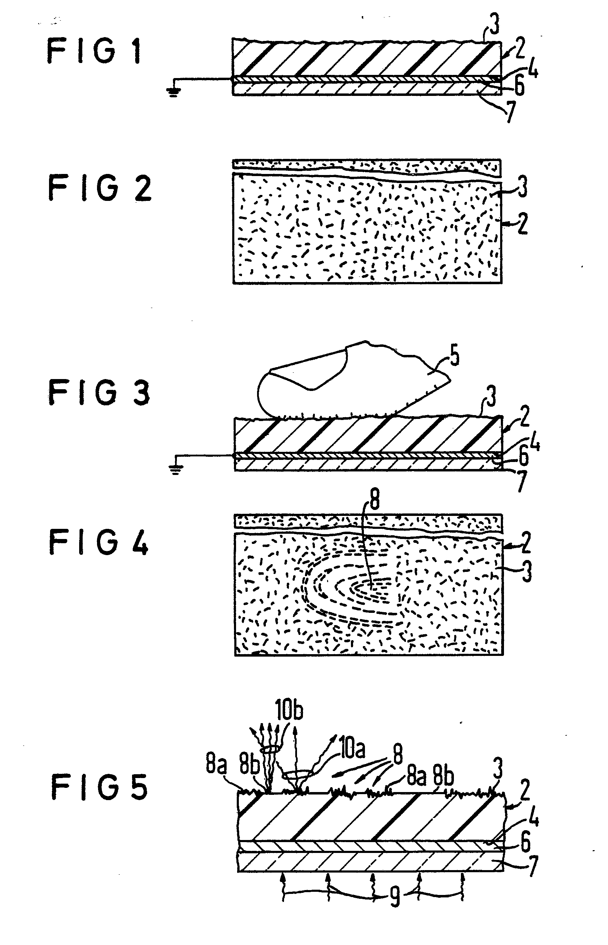

Gemäß der Figur 1 ist als Abtastelement 2 eine rechtwinkelige flache Platte vorgesehen. Das Abtastelement 2
weist eine erste Fläche, genannt Abtastfläche 3, und
eine zweite Fläche, genannt untere Fläche 4, auf. Das
Abtastelement 2 ist aus Material gefertigt, welches
wiederbenutzbar ist, vorzugsweise aus einem elastomerischen oder thermoplastischen Material, beispielsweise plastifiziertes Polystyrol. Das Material des
Abtastelements 2 ist elektrisch nicht leitend. Es ist
so ausgewählt, daß seine Übergangs- oder Erweichungstemperatur höher als die das Element zwei umgebende
Umgebungstemperatur und in der Nähe der Temperatur des
menschlichen Körpers liegt. Insbesondere sollte die Erweichungstemperatur so gewählt sein, daß sie größer als
etwa 40°C ist. Sie kann so gewählt sein, daß sie nur
einige wenige Celsiusgrade über der Umgebungstemperatur
liegt. Die Abtastfläche 3 ist so ausgebildet, daß sie
für eine kurze Zeitdauer den Druck eines in Figur 3 dargestellten, zu untersuchenden Fingers 5 aufnimmt. Allgemein gesprochen ist die Erweichungstemperatur so ausgewählt, daß eine dünne Schicht des Materials der Abtastfläche 3 zu schmelzen beginnt, wenn der Finger 5 draufgedrückt wird. Abweichend von der Darstellung nach
Figur 1 braucht die Abtastfläche 3 des Abtastelements
2 nicht flach zu sein. Sie kann auch derartig gekrümmt
sein, daß sie dem Umriß des Fingers 5 angepaßt ist.

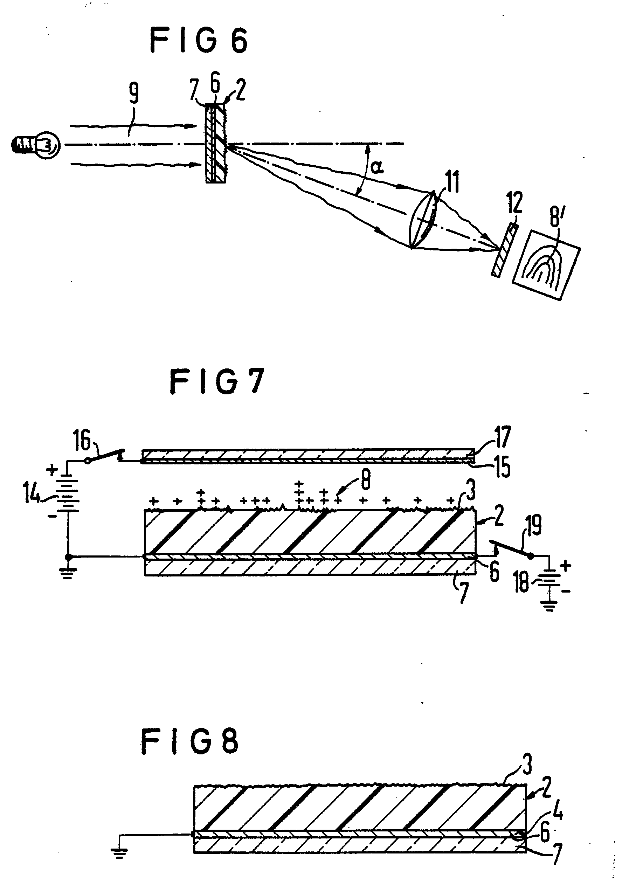

An der zweiten Fläche 4 ist eine Elektrode 6 angebracht.
Die Elektrode 6 ist bevorzugterweise aus einem Material
gefertigt, welches im Hinblick auf das Licht eines
abfragenden Strahls, der in den Figuren 5 und 6 angedeutet ist, transparent ist. Sie kann aus Indiumoxid
(InO) gefertigt sein. Die Elektrode 6 ist in der Form
einer dünnen rechtwinkeligen Platte oder eines entsprechenden Drahtsystems ausgebildet, oder kann als
dünner Filmüberzug ausgebildet sein. Die Elektrode 6
kann geerdet sein. Sie ist von einer lichtdurchlässigen Trägerplatte 7 getragen, beispielsweise von einem Glassubstrat.

Gemäß den Figuren 1 und 2 weist die Abtastfläche 3 ein
Oberflächenrelief zufälliger Konfiguration auf. In
anderen Worten ausgedrückt, die Oberfläche 3 enthält
viele kleine Erhebungen und/oder Vertiefungen in einer
Zufallsverteilung. Diese Variationen in den Erhebungen
in oder auf der Fläche, die kein spezielles Muster aufweisen, können vorzugsweise auf eine weiter unten näher
beschriebene Art und Weise erzeugt werden. Bei Belichtung der Fläche 3 würden alle Segmente das Licht in
einer zufälligen Weise streuen, so daß keine bevorzugte
Streurichtung vorläge. Die Erzeugung eines dünnen Oberflächenreliefs zufälliger Konfiguration bildet einen
ersten Verfahrensschritt.

In einem zweiten Verfahrensschritt wird der Finger 5
gegen die flache Abtastfläche 3 des Abtastelements 2
gedrückt. Dies kann der Figur 3 entnommen werden.

Der nächste Verfahrensschritt liegt in der Wegnahme des
Fingers 5 von der Abtastfläche 3. Wie in der Figur 4
dargestellt, ist ein Fingerabdruck 8 auf der Fläche 3
zurückgelassen worden. Aufgrund des bzw. der auf die
Oberfläche 3 durch die Erhebungen des Fingers ausgeübten
Druckes bzw. Temperatur schmilzt das zufällige Oberflächenrelief lokal. In den geschmolzenen Abschnitten
existieren keine Erhebungen und keine Vertiefungen mehr.
Diese Abschnitte sind "ausgebügelt"; sie bilden jetzt
flache Bereiche.

Dies ist in der Figur 5 dargestellt. Bereiche oder Abschnitte, in welchen noch eine statistische Verteilung
vorhanden ist, sind mit 8a und Bereiche oder Abschnitte,
in denen die statistische Verteilung der kleinen Vertiefungen und/oder Erhebungen durch die Erhebungen des
Fingers "ausgebügelt"worden sind, sind mit 8b bezeichnet.

In den Figuren 5 und 6 ist der nächste Verfahrensschritt dargestellt. In diesem Schritt wird die in dem "eingefrorenen" Fingerabdruck enthaltene Information optisch ausgelesen und analysiert. Dies kann mittels des Lichts eines abfragenden oder lesenden Strahls 9 entweder in Transmission oder in Reflexion ausgeführt werden. Die Ausführungsform nach den Figuren 5 und 6 zeigt ein Auslesen mittels transmittierenden Lichts. Der Strahl 9 wird senkrecht auf die untere Fläche 4 des Abtastelements 2 gerichtet. Zu diesem Zweck wird der Strahl 9 durch das transparente Element 7 und die transparente Elektrode 6 in das Abtastelement 2 transmittiert. Schließlich erreicht es die obere Fläche 3. Nachdem es den Fingerabdruck 8 passiert hat, trägt es die Fingerabdruckinformation. Das Licht wird entsprechend den Vertiefungen und Erhöhungen der Fläche 3 räumlich moduliert, d.h. es trägt die Information über die geometrische Struktur des Fingerabdrucks 8. Die Analysierung der in dem modulierten Licht enthaltenen Information kann prinzipiell auf irgend eine bekannte Art und Weise ausgeführt werden. In den Figuren 5 und 6 ist jedoch eine sehr einfache Methode veranschaulicht.

In den Abschnitten 8b wird das Licht nur in engen Kegeln 10a gestreut, welche die optische Achse des illuminierenden Strahls umgeben (Vorwärtsstreuung). In den Abschnitten 8a jedoch, in denen die zufällige Verteilung des ursprünglichen Oberflächenreliefs durch die Erhöhungen des berührenden Fingers nicht geändert worden ist, wird der illuminierende Strahl in weit geöffneten Kegeln 10a gestreut. Das bedeutet, daß die geschmolzenen Teile des Elements 2 nicht mehr länger Licht in der gleichen Weise streuen als in der unzerstörten Umgebung. Gemäß Figur 6 kann das Fingerabdruckmuster in Form von dunklen Linien auf einem erleuchteten Hintergrund gesehen werden, wenn die Fläche 3 unter einem spitzen Winkel $\alpha$ relativ zur Normalen gesehen wird. Das Bild der Abtastfläche 3 und das Bild 8' des Fingerabdrucks 8 kann über ein optisches System 11 auf eine lichtempfindliche Vorrichtung 12 gerichtet werden. Diese Vorrichtung 12 kann aus einem Feld aus Fotoelektroden zum Umformen des Bildes in elektrische Signale bestehen. Wie aus der Figur 7 hervorgeht, wird im nächsten Schritt des Verfahrens der Fingerabdrucksensor in seinen ursprünglichen Zustand zurückgesetzt. Dieser Schritt umfaßt eine elektrostatische Aufladung und gleichzeitige Erwärmung. Zu diesem Zweck sind eine Einrichtung zum elektrostatischen Aufladen und eine Heizeinrichtung vorgesehen. Die Einrichtung zum Aufladen umfaßt eine Spannungsquelle 14 mit einer Hochspannung V, die mittels eines Schalters 16 zwischen Masse und einer Elektrode 15 geschaltet werden kann. Die Elektrode 15 ist in einem Abstand von der Abtastfläche 3 angeordnet. Sie wird von einem Trägerelement 17 getragen, beispielsweise von einem Glassubstrat. Die Elektrode 15 kann aus einem einzigen Draht bestehen, der parallel zur Fläche 3 angeordnet ist. In der vorliegenden Ausführungsform besteht sie aus einer planen Elektrode, die mit der unteren Fläche des Trägerelements 17 verbunden ist.

Sobald der Schalter 16 geschlossen worden ist, bewirkt die Hochspannung V zwischen den Elektroden 6 und 15 eine Ionisierung der in der Nähe der Elektrode 15 befindlichen Luft. Aufgrund der Ionisierung der Luft werden elektrische Ladungen zu der Abtastfläche 3 gelenkt. Diese Ladungen werden auf der Fläche 3 gehalten. Sie können von der Fläche 3 nicht abfließen, weil das Abtastelement 2 einen elektrischen Isolator bildet. Wie ebenfalls in der Figur 7 dargestellt, ist der positive Anschluß der Hochspannungsquelle 14 mit der Elektrode 15 verbunden, während der negative Anschluß mit der Elektrode 6 verbunden ist. Deshalb wird die Fläche 3 mit positiven Teilchen aufgeladen. Eine Aufladung mit negativen Teilchen ist jedoch ebenfalls

möglich. Um eine negative elektrostatische Aufladung zu erhalten, ist es lediglich erforderlich, die Polarität der Hochspannungsquelle 14 in Bezug auf die Elektroden 6 und 15 umzukehren. Wie es in der Figur 7 angedeutet ist, würden sich die positiven Partikel ohne Erwärmung in gewissen Flächenelementen im Bereich des Fingerabdrucks 8 sammeln. Aufgrund der lokal variierenden Ladungsdichte wären unterschiedliche elektrische Feldstärken vorhanden und unterschiedliche elektrische Kräfte würden auf verschiedene Flächenelemente ausgeübt.

In der Figur 7 ist auch eine Heizeinrichtung dargestellt, welche in der vorliegenden Ausführungsform elektrischer Natur ist, insbesondere aus einer Widerstands-Heizeinrichtung besteht. Die Heizeinrichtung besteht aus einer Heizstromquelle 18 und einem Schalter 19. Die Heizstromquelle 18 und der Schalter 19 sind zwischen einem Ende der Elektrode 6 und Masse in Serie geschaltet. Das andere Ende der Elektrode 6 ist ebenfalls mit Masse verbunden. Wenn der Schalter 19 geschlossen wird, liefert die Heizstromquelle 18 einen Heizstrom (Gleichstrom oder pulsierenden Strom), der durch die Elektrode 6 fließt. Demgemäß ist die Elektrode 6 nicht nur eine Hochspannungselektrode, die zur elektrostatischen Aufladung benutzt wird, sie ist zugleich ein Heizwiderstand, beispielsweise ein Widerstandsgitter. Der Heizstrom heizt das Abtastelement 2 auf. Die Größe des Heizstroms ist so gewählt, daß auf der Abtastfläche 3 eine Temperatur erreicht wird, die wenigstens näherungsweise der Erweichungstemperatur des elastomerischen oder thermoplastischen Materials entspricht. Im Laufe des Erwärmungsvorgangs wird die Fläche 3 des Abtastelements 2 weich und beginnt zu schmelzen. Jetzt kann die Fläche 3 flach werden oder sich glätten und der Fingerabdruck 8 wird ausgelöscht. Die elektrostatische Ladung verteilt sich zufällig auf der Fläche, wodurch elektrostatische Kräfte auf die Fläche 3 ausgeübt werden, die eine Zufallsverteilung von Vertiefungen und/
oder Erhebungen zur Folge haben. Die Folge davon ist,
daß wieder ein Wandler erhalten wird, der statistische
Oberflächeneigenschaften aufweist. Dieser Effekt ist
in der Literatur als Frost-Effekt thermoplastischer
Medien bekannt.

Gemäß Figur 8 liegt der letzte Verfahrensschritt im
Abkühlen des Abtastelements 2. Dies kann einfach durch
Ausschalten des Schalters 19 erreicht werden, vorausgesetzt, daß die Umgebungstemperatur niedrig genug ist.
Zusätzlich kann eine nicht dargestellte Kühleinrichtung
an das Abtastelement 2 angeschlossen sein, beispielsweise thermoelektrische Peltier-Elemente. Das Abkühlen
des Abtastelements 2 hat ein "Einfrieren" des Zufallmusterreliefs der Fläche 3 zur Folge. Es sei darauf
hingewiesen, daß während des Abkühlvorganges die
Elektrode 15 von der Hochspannungsquelle 14 abgeschaltet ist. Nach dem Abkühlen ist das Abtastelement
2 für den nächsten Fingerabdruckvorgang und Lesezyklus
bereit.

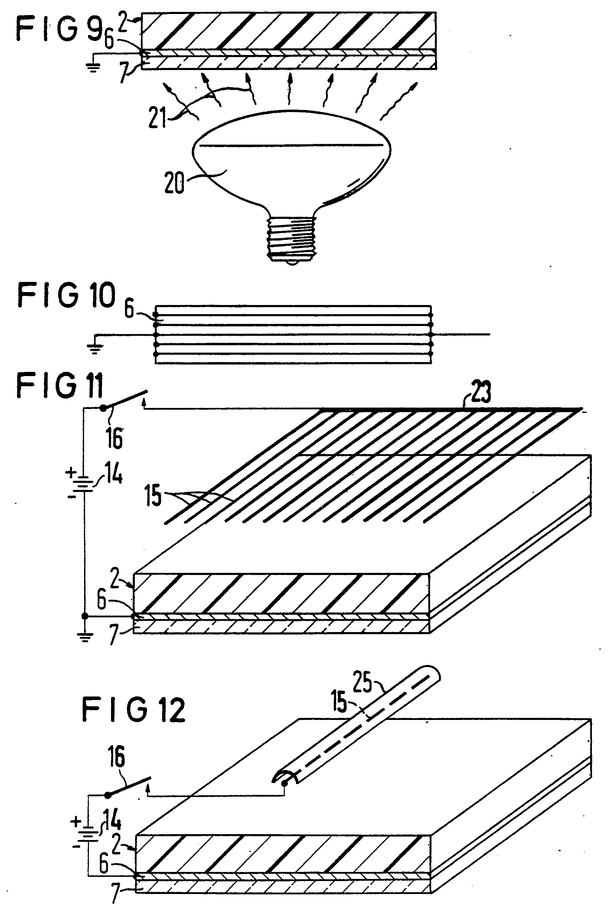

Aus Figur 9 geht hervor, daß das Erwärmen des Abtastelements 2 (siehe Figur 7) auch mittels einer strahlenden
Heizeinrichtung erreicht werden kann. In diesem Fall wird
eine IR-Strahlungsquelle 20 benutzt, welche IR-Strahlung
21 in Richtung der unteren Seite der Elektrode 6 sendet.
Die IR-Quelle 20 kann eine einzige IR-Quelle oder ein
Feld aus einzelnen IR-Quellen sein. Als IR-Quelle 20
kann eine IR-Strahlung imitierende LED oder eine IR-
Lampe benutzt werden.

Aus der Figur 10 geht eine Ausführungsform der Elektrode
6 hervor. Die Elektrode 6 kann aus einem Feld paralleler
Widerstandsdrähte bestehen. Diese Drähte können sowohl
als Elektrode beim elektrostatischen Aufladungsvorgang
als auch als Heizkörper beim Erwärmungsvorgang dienen.

In der Figur 11 ist eine perspektivische Darstellung einer anderen Ausführungsform der Erfindung dargestellt. Bei dieser Ausführungsform besteht die Elektrode 15 zum Aufladen der oberen Fläche des Elements aus einem Feld paralleler Dränte, die auf einer Seite an ein Verbindungselement 23 angeschlossen sind, welches seinerseits über den Schalter 16 mit der Hochspannungsquelle 14 verbunden ist. Das Feld paralleler Drähte kann durch geeignete Einrichtungen, welche nicht dargestellt sind, gehalten sein.

In der Figur 12 ist eine perspektivische Darstellung einer noch anderen Ausführungsform der Erfindung gezeigt. Bei dieser Ausführungsform besteht die Elektrode 15 aus einem einzigen Draht, der parallel zur Fläche des Elements 2 gehalten ist. Die Elektrode 15 ist auf der von der Fläche 3 abgewandten Seite durch einen Schirm 25 abgeschirmt. Deshalb werden geladene Teilchen im wesentlichen zwischen dem Schirm 25 und der oberen Fläche 3 des Abtastelements 2 erzeugt.

12 Patentansprüche

12 Figuren

Patentansprüche

1. Fingerabdruckwandler mit einem Abtastelement (2), das eine Abtastfläche (3) zum Aufdrücken eines Fingers (5) aufweist, d a d u r c h  g e k e n n z e i c h n e t , daß das Abtastelement (2) aus einem Material gefertigt ist, das eine Erweichungstemperatur aufweist, die höher ist als die Umgebungstemperatur und die in der Nähe der Temperatur des Fingers (5) liegt, so daß das Material zu schmelzen beginnt, wenn der Finger (5) auf die Abtastfläche (3) gedrückt wird, und daß die Abtastfläche (3) ein zufällig verteiltes Oberflächenrelief aufweist.

2. Wandler nach Anspruch 1, d a d u r c h  g e k e n n z e i c h n e t , daß das Oberflächenrelief zufälliger Verteilung Erhebungen umfaßt, die aus dem gleichen Material sind, wie das Abtastelement (2).

3. Wandler nach Anspruch 1 oder 2, d a d u r c h  g e k e n n z e i c h n e t , daß eine Einrichtung (6, 14, 16, 15) zum Erzeugen einer elektrostatischen Aufladung der Abtastfläche (3) und eine Einrichtung (6, 19, 18; 20) zum Erwärmen der Abtastfläche (3) des Abtastelements (2) auf eine Temperatur vorgesehen sind, die wenigstens der Erweichungstemperatur des Materials entspricht, aus dem das Abtastelement (2) gefertigt ist, wodurch ein Fingerabdruck auf der Abtastfläche (3) gelöscht und das Relief durch Erwärmen der genannten Fläche und gleichzeitiger Erzeugung der genannten Aufladung in seiner zufälligen Form wiederherstellbar ist.

4. Wandler nach einem der vorhergehenden Ansprüche, d a d u r c h  g e k e n n z e i c h n e t , daß das Abtastelement (2) aus einer flachen Platte besteht, die eine erste Seite (3) und eine zweite Seite (4) aufweist, welche Seiten (3, 4) im wesentlichen parallel zueinander sind, wobei die erste Seite (3) die besagte Abtastfläche bildet und wobei an der zweiten Seite (4) eine Elektrode (6) angebracht ist, die für einen abfragenden Lichtstrahl wenigstens teilweise transparent ist.

5. Wandler nach einem der vorhergehenden Ansprüche, d a d u r c h   g e k e n n z e i c h n e t , daß das Material des Abtastelements (2) aus einem elastomerischen Material besteht.

6. Wandler nach Anspruch 5, d a d u r c h   g e - k e n n z e i c h n e t , daß das elastomerische Material aus plastifiziertem Polystyrol besteht.

7. Wandler nach einem der vorhergehenden Ansprüche, d a d u r c h   g e k e n n z e i c h n e t , daß das Material des Abtastelements (2) aus einem thermoplastischen Material besteht.

8. Wandler nach einem der vorhergehenden Ansprüche, d a d u r c h   g e k e n n z e i c h n e t , daß eine Lichtquelle zum Aussenden von Licht in Richtung der Abtastfläche (3) vorgesehen ist, daß das Material des Wandlers in Bezug auf dieses Licht transparent ist, und daß eine lichtempfindliche Einrichtung (12) zum Erzeugen eines Bildes der Abtastfläche (3) vorgesehen ist, die in einem Winkel ( $\alpha$ ) zur Normalen der Abtastfläche (3) angeordnet ist.

9. Wandler nach Anspruch 8, d a d u r c h   g e - k e n n z e i c h n e t , daß die lichtempfindliche Einrichtung (12) ein Fotoelektrodenfeld zum Umwandeln des Bildes in elektrische Signale umfaßt.

10. Verfahren zur Analysierung eines Fingerabdrucks, g e k e n n z e i c h n e t   d u r c h   folgende

0040838

Verfahrensschritte:

a) es wird ein Oberflächenrelief zufälliger Verteilung auf einer Abtastfläche (3) eines Abtastelements (2) erzeugt, wobei das Abtastelement (2) aus einem Material gefertigt ist, welches eine Erweichungstemperatur aufweist, die höher als die Umgebungstemperatur und in der Nähe der Temperatur eines menschlichen Fingers liegt;

b) es wird auf der Abtastfläche (3) durch Aufdrücken des Fingers (5) ein Fingerabdruck (8) erzeugt, wobei das Material des Abtastelements (2) partiell schmilzt; und

c) der Fingerabdruck (8) wird von der Abtastfläche (3) optisch ausgelesen.

11. Verfahren nach Anspruch 10, g e k e n n z e i c h n - n e t   d u r c h   folgende weitere Verfahrensschritte:

d) die Abtastfläche (3) wird elektrostatisch aufgeladen;

e) die Abtastfläche (3) wird gleichzeitig auf eine Temperatur erwärmt, die wenigstens der Erweichungstemperatur des Materials des Abtastelements (2) entspricht; und

f) das Abtastelement (2) wird danach auf eine Temperatur abgekühlt, die unterhalb der Erweichungstemperatur liegt.

12. Verfahren nach Anspruch 11, d a d u r c h   g e - k e n n z e i c h n e t , daß die Abtastfläche (3) auf die Umgebungstemperatur abgekühlt wird.

0040838

FIG 1

FIG 2

FIG 3

FIG 4

FIG 5

FIG 6

FIG 7

FIG 8

0040838

80P 8217 DE

FIG 9

FIG 10

FIG 11

FIG 12

**Europäisches Patentamt**

# EUROPÄISCHER RECHERCHENBERICHT

EP 81 10 3955

| | EINSCHLÄGIGE DOKUMENTE | | KLASSIFIKATION DER ANMELDUNG (Int. Cl.3) |
|---|---|---|---|
| **Kategorie** | **Kennzeichnung des Dokuments mit Angabe, soweit erforderlich, der maßgeblichen Teile** | **betrifft Anspruch** | |
| | US - A - 3 532 426 (C.G. LEMMOND) <br> * Zusammenfassung; Spalte 4, Zeile 9 - Spalte 5, Zeile 37; Spalte 6, Zeilen 6-16; Abbildungen * <br><br> -- <br><br> GB - A - 900 068 (GENERAL ELECTRIC) <br> * Patentansprüche; Abbildung 1; Seite 2, Zeile 40 - Seite 3, Zeile 110 * <br><br> -- | 1,7- 11 <br><br><br><br><br> 1,3,4, 7,11, 12 | A 61 B 5/10 |
| A | FR - A - 2 448 338 (ENERGY CONVERSION DEVICES) <br> * Seite 8, Zeile 20 - Seite 13, Zeile 21; Patentansprüche; Abbildungen * <br><br> -- | 1 | **RECHERCHIERTE SACHGEBIETE (Int. Cl.3)** <br><br> A 61 B 5/10 <br> G 07 C 11/00 <br> G 06 K 9/00 <br> 9/20 <br> G 03 G 5/022 <br> B 41 M 5/26 |
| A | US - A - 3 533 823 (W.H. NEWKIRK) <br> * Zusammenfassung; Abbildungen * <br><br> -- | 1 | |
| A | DE - A - 27 304 (H. MAU) <br> * Patentanspruch * <br><br> ---- | 1 | **KATEGORIE DER GENANNTEN DOKUMENTE** <br><br> X: von besonderer Bedeutung <br> A: technologischer Hintergrund <br> O: nichtschriftliche Offenbarung <br> P: Zwischenliteratur <br> T: der Erfindung zugrunde liegende Theorien oder Grundsätze <br> E: kollidierende Anmeldung <br> D: in der Anmeldung angeführtes Dokument <br> L: aus andern Gründen angeführtes Dokument <br> &: Mitglied der gleichen Patentfamilie, übereinstimmendes Dokument |

Der vorliegende Recherchenbericht wurde für alle Patentansprüche erstellt.

| Recherchenort | Abschlußdatum der Recherche | Prüfer |
|---|---|---|
| Den Haag | 01-09-1981 | DAVID |

EPA form 1503.1   06.78